(19) **Europäisches Patentamt**

**European Patent Office**

**Office européen des brevets**

(11) **EP 1 318 775 B1**

(12) **EUROPEAN PATENT SPECIFICATION**

(45) Date of publication and mention
of the grant of the patent:
**29.11.2006 Bulletin 2006/48**

(51) Int Cl.:
***A61F 2/24*** (2006.01)

(21) Application number: **01971151.4**

(22) Date of filing: **19.09.2001**

(86) International application number:
**PCT/US2001/029129**

(87) International publication number:
**WO 2002/024119 (28.03.2002 Gazette 2002/12)**

(54) **VALVED PROSTHESES WITH REINFORCED POLYMER LEAFLETS**

VENTILPROTHESEN MIT BLATTELEMENTEN AUS VERSTÄRKTEM KUNSTSTOFF

PROTHESES A VALVES COMPORTANT DES VALVES POLYMERES RENFORCEES

(84) Designated Contracting States:
**AT BE CH CY DE DK ES FI FR GB GR IE IT LI LU MC NL PT SE TR**

(30) Priority: **21.09.2000 US 666823**

(43) Date of publication of application:
**18.06.2003 Bulletin 2003/25**

(73) Proprietor: **ST. JUDE MEDICAL, INC.**
**St. Paul, MN 55117 (US)**

(72) Inventors:
• **WOO, Yi-Ren**
**Woodbury, MN 55129 (US)**
• **CAI, Chad, O.**
**Woodbury, MN 55125 (US)**
• **KURK, James, L.**
**Anoka, MN 55014 (US)**
• **KRUSE, Steven, D.**
**St. Micheal, MN 55376 (US)**

(74) Representative: **Wakerley, Helen Rachael**
**Reddie & Grose,**
**16 Theobalds Road**
**London WC1X 8PL (GB)**

(56) References cited:
| | |
|---|---|
| EP-A- 0 850 607 | WO-A-01/05334 |
| US-A- 3 717 883 | US-A- 4 222 126 |
| US-A- 4 556 996 | US-A- 4 731 074 |
| US-A- 4 778 461 | US-A- 5 139 515 |

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

**Description**

BACKGROUND OF THE INVENTION

The invention relates to valved prostheses with leaflets formed from polymers. In particular, the invention relates to valved prostheses, especially heart valve prostheses, with reinforced polymer valve leaflets.

Heart valve insufficiency can be a debilitating and possibly life threatening condition. For example, heart valve regurgitation, i.e., backward leakage of blood at a heart valve, results in reduced pumping efficiency. In addition, pumping inefficiency and pooling of blood in extremities can result from insufficiency of valves in veins.

With respect to mitral valve regurgitation, compensatory mechanisms such as hypertrophy and dilation of the ventricle suggest early treatment to prevent progressive deterioration of ventricular function. Diagnosis of mitral regurgitation can be performed using visualization with transesophageal echocardiography or by echocardiography. In particular, defective leaflet coaptation and the site and direction of the regurgitant flow can be examined to evaluate likely modes of failure. Similar approaches can be used to evaluate insufficiency of heart valves in other positions, such as the aortic position.

Some cases of heart valve regurgitation can be repaired by modifications of the original valve in a procedure generally referred to as valvuloplasty. For example, one repair technique uses an annuloplasty ring to provide structural support to the natural annulus of the native valve. For severe cases of heart valve damage or disease, however, reconstructive valvular surgery may not be possible. In such cases, valve replacement may be indicated.

Physicians use a variety of prostheses to correct problems associated with the cardiovascular system, especially the heart. For example, the ability to replace or repair damaged or diseased heart valves with prosthetic devices has provided surgeons with a method of treating heart valve deficiencies due to disease and congenital defects. A typical procedure involves removal of the native valve and surgical replacement with a prosthetic heart valve.

Prosthetic heart valve leaflets or occluders perform the function of opening and closing to regulate the blood flow through the heart valve. Typically, heart valve leaflets must either pivot or flex with each cycle of the heart to open and close. Heart valves function as check valves, which open for flow in one direction and close in response to pressure differentials.

Prostheses can be constructed from natural materials such as tissue, synthetic materials or a combination thereof. Prostheses formed from purely synthetic materials can be manufactured, for example, from biocompatible metals, ceramics, carbon materials, such as graphite, polymers, such as polyester, and combinations thereof. Heart valve prostheses with purely synthetic materials can be manufactured with rigid occluders or leaflets that pivot to open and close the valve, or flexible leaflets that flex to open and close the valve.

Although mechanical heart valves with rigid pivoting occluders have the advantage of proven durability through decades of use, they are associated with blood clotting on or around the prosthetic valve. Blood clotting can lead to acute or subacute closure of the valve or associated blood vessel. For this reason, patients with implanted mechanical heart valves remain on anticoagulants for as long as the valve remains implanted. Anticoagulants have associated risks, such as hemorrhages, and cannot be taken safely by certain individuals.

Heart valve prostheses with flexible leaflets can be constructed with tissue leaflets or polymer leaflets. Prosthetic tissue heart valves can be derived from, for example, porcine heart valves or manufactured from other biological material, such as bovine pericardium. In prostheses with flexible leaflets, the leaflets are generally designed to approximate natural leaflet function. While the leaflets are flexible, they must have a well defined and stable configuration to properly close the valve at each cycle in response to pressure differentials. Also, the leaflets should be durable to provide stable performance over many years of use.

Biological materials in prosthetic heart valves generally have a profile that produces less turbulent blood flow. Therefore, intravascular clotting is less likely to occur than with mechanical heart valves. Tissue based bioprostheses do not require the long term use of anticoagulants due to a lower incidence of thromboembolism. While tissue leaflets have desired flexibility and acceptable hemodynamic performance, tissue leaflets can calcify after implantation, which may result in loss of flexibility, resulting in improper closure and/or opening of the valve. While polymer leaflets can be incorporated into heart valve prostheses, these polymers should provide long term stable function to be suitable alternatives for tissue leaflets or pivoting mechanical leaflets.

Document US-A-4 222 126 discloses a valve prosthesis according to the preamble of claim 1.

SUMMARY OF THE INVENTION

The invention pertains to a valve prosthesis according to claim 1.

BRIEF DESCRIPTION OF THE DRAWINGS

Fig. 1 is a perspective view of a heart valve prosthesis with reinforced polymer leaflets, wherein the valve is in an open configuration.
Fig. 2A is a sectional view of one embodiment of a reinforced polymer leaflet with a single reinforcement at the coaptation edge, the cross section being taken along the line 2-2 of Fig. 1.

Fig. 2B is a sectional view of an alternative embodiment of a reinforced polymer leaflet with a single reinforcement, the cross section taken along the line 2-2 of Fig. 1.

Fig. 2C is a sectional view of an alternative embodiment of a reinforced polymer leaflet with a different material located at the free edge, the cross section being taken along the line 2-2 of Fig. 1.

Fig. 2D is a sectional view of an alternative embodiment of a reinforced polymer leaflet with a plurality of reinforcements taken along the line 2-2 of Fig. 1.

Fig. 2E is a sectional view of an alternative embodiment of a reinforced polymer leaflet with a plurality of reinforcements taken along the line 2-2 of Fig. 1.

Fig. 2F is a sectional view of an alternative embodiment of a reinforced polymer leaflet with a reinforcing polymer layer taken along the line 2-2 of Fig. 1.

Fig. 2G is a sectional view of an alternative embodiment of a reinforced polymer leaflet with a partial reinforcing polymer layer taken along the line 2-2 of Fig. 1.

Fig. 2H is a sectional view of an alternative embodiment of a reinforced polymer leaflet with an embedded reinforcing polymer layer taken along the line 2-2 of Fig. 1.

Fig. 2I is a sectional view of an alternative embodiment of a reinforced polymer leaflet with an embedded partial reinforcing polymer layer taken along the line 2-2 of Fig. 1.

Fig. 3 is a perspective view of a heart valve prosthesis with reinforced polymer leaflets, wherein the valve is in a closed configuration.

Fig. 4 is a side view of the prosthesis of Fig. 3.

Fig. 5 is a sectional view of the prosthesis of Fig. 3 taken along line 5-5 in Fig. 3.

Fig. 6A is a sectional view of one embodiment of two adjacent reinforced polymer leaflets showing the coaptation region.

Fig. 6B is a sectional view of an alternative embodiment of two adjacent reinforced polymer leaflets showing the coaptation region.

Fig. 7 is a side view of an embodiment of a polymer leaflet with a reinforcement at the center of the free edge.

Fig. 8 is a side view of an embodiment of a polymer leaflet with reinforcements at the sides of the free edge.

Fig. 9 is a fragmentary perspective view of a vascular prosthesis incorporating a valve having polymer leaflets in which a portion of the prosthesis has been removed to expose the valve.

Fig. 10 is a fragmentary side view of a left ventricular assist device with polymer valves, in which the sides of the inflow and outflow tubes have been removed to expose the inflow and outflow valves.

## DETAILED DESCRIPTION OF THE ILLUSTRATIVE EMBODIMENTS

Improved polymer leaflets for valved prostheses, especially heart valve prostheses, include reinforcements along the coaptation edge of the leaflet to inhibit tearing and other damage or wear of the polymer at the free edge. Using the approaches described herein, a polymer leaflet less susceptible to damage or wear can be produced without compromising the performance of the leaflet in a prosthesis. In some preferred embodiments, the reinforcement includes a fiber at or near the coaptation edge. In other preferred embodiments, the leaflets have a uniform composition across the leaflet except for a reinforcement at or near the coaptation edge. In alternative embodiments, the reinforcement extends as a layer through all or a significant portion of the extent of the leaflet. The resulting valve can have excellent hemodynamic properties with reduced likelihood of damage or wear during use.

Damaged or diseased native heart valves can be replaced to restore valve function. Heart valve prostheses of interest have leaflets formed from polymers. The polymers form flexible leaflets similar in structure to native tissue leaflets. The polymer heart valve prosthesis can be designed as a replacement for any heart valve, i.e., an aortic valve, a mitral valve, a tricuspid valve, or a pulmonary valve. In addition, the improved polymer valve prostheses can be used for the replacement of vascular valves. The patient can be an animal, especially a mammal, and preferably is a human.

The base of the support structure anchors the valve and provides support for the fixed lower portion of the valve. In a flexible valve, the leaflets are supported by a support structure that includes commissure supports and scallops between the commissure supports. The base of the valve may include a sewing cuff or the like for attachment of the valve to the patient's annulus or to the other components of the device.

In some embodiments, the support structure includes a rigid component that maintains the leaflet function of the valve against the forces opening and closing the valve in use. Valves with a rigid support structure are termed stented valves, and the rigid support is called a stent. The stent provides a scaffolding for the leaflets. The stent includes commissure supports that support the ends of the free edge of the leaflets. Scallops, which support the attached edges of the leaflets, extend between the commissure supports. The stent generally is sufficiently rigid such that only the base of the stent is attached to the patient or other device. As a particular example, heart valve stents are used to support leaflet components within a prosthetic heart valve.

In alternative embodiments, the support structure is not sufficiently rigid to maintain the leaflet function of the valve against the forces opening and closing the valve. In these embodiments, the valve is termed stentless. In a stentless valve, a structure with commissure supports and scallops supports the attached edge of the leaflets. However, in the stentless valve, the leaflet support structure is less rigid such that the entire support structure must be secured to other anatomical structures, such as the wall of a blood vessel, to prevent the valve from collapsing against the fluid pressure. If the support structure is supported by attachment to other structures, the leaflets will have proper coaptation. The support structure can comprise the polymer of the leaflets or other flexible material in a generally cylindrical configuration that defines the commissure supports and the scallops that hold the edges of the leaflet. The flexible support structure generally is sutured or otherwise attached to the wall of the corresponding blood vessel or other structure.

The polymer leaflets are configured to flex in response to changes in blood flow. In particular, preferred embodiments of the valves function as one way check valves that open to allow flow in a desired direction and close in response to pressure differentials. Thus, when blood is flowing downstream, the leaflets fully open to reduce resistance to the blood flow. In their open configuration, the leaflets open to a nearly cylindrical shape to allow for flow through the valve.

When cyclic pumping produces higher pressure downstream or lower pressure upstream, the valve closes in response to pressure differentials. The reinforced edges of adjacent leaflets contact in the closed configuration with the leaflets extending across the lumen. The mated portion of the leaflets is referred to as the coaptation region.

The free edge of the polymer leaflet may be subject to tearing or similar damage or wear during use or during implantation. If the edge tears, the valve may leak in a back flow direction when the valve is closed. During use, the tear can deteriorate further. The reinforcement of the free edge can reduce the incidence of tearing or other damage or wear to the free edge without affecting the mechanical performance of the valve. When the valve is closed, the reinforced edge will form at least a portion of the coaptation region.

The reinforcement of free edges of the leaflets can be formed from either a modification of the polymer composition, a reinforcing member, such as a fiber or fabric, embedded in the polymer forming the face of the leaflet, or by a thickened portion of a polymer located at or near the edge of the leaflet. In preferred embodiments, the leaflet area away from the free edge has a uniform composition of flexible polymer. In these embodiments, the reinforcement of the free edge has little, if any, effect on the performance of the valve with respect to efficient opening and closing of the valve. In alternative embodiments, the body of the leaflet can include other reinforcements away from the edge, for example, in the form of either modified polymer compositions, reinforcing members and/or thickened polymer portions. Reinforcements away from the free edge can improve the durability of the valve.

In other embodiments, the reinforcement has the form of a thin layer of a second material, such as a polymer film or a fabric, that is stronger than the primary polymer material forming the remaining portion of the leaflet. This reinforcing layer can be sandwiched inside of two layers of a primary polymer material, or the reinforcing layer can be a layer on the surface of the primary polymer. Generally, the reinforcing layer has an area at least about 50 percent of the area of the leaflet and can extend over the entire leaflet.

The leaflets are formed from a thin film of flexible polymer. Suitable polymers are biocompatible, in that they are non-toxic, non-carcinogenic and do not induce hemolysis or an immunological response. Heart valve prostheses formed from polymers preferably are non-thrombogenic. Relevant mechanical properties of polymers include, for example, stiffness, strength, creep, hardness, fatigue resistance and tear resistance. Preferred polymers are durable in that they do not significantly lose their flexibility and do not significantly lose their mechanical strength following many years of use.

The leaflets can be formed by a variety of casting and molding processes. In preferred embodiments, the leaflets are formed by dip coating a mandrel. Generally, the leaflets are formed directly in association with the corresponding leaflet support structure, either a stent or a flexible support structure.

Valved Prostheses

The improved reinforced polymer leaflets can be used in valved prostheses. In particular, the leaflets can be used in artificial hearts, heart valve prostheses, valved vascular prostheses or left ventricular assist devices. The polymer leaflets open and close to control flow through the valve. The free edges are reinforced to reduce or eliminate tearing or other damage or wear to the edge of the leaflet.

Heart valve prostheses with polymer leaflets are suitable for the replacement of damaged or diseased native heart valves. While the embodiments of the heart valve prosthesis shown in the figures below have three polymer leaflets, heart valve prostheses can be constructed with different numbers of polymer leaflets, such as two leaflets, four leaflets or more than four leaflets. The prosthesis may or may not have the same number of leaflets as the natural valve that it is used to replace.

Mammalian veins include valves that assist with blood circulation by limiting the amount of back flow in the veins. Veins collect blood from capillaries and are responsible for returning blood to the heart. Generally, vascular valves are replaced as part of a vascular graft with sections of conduit.

Mammalian hearts have four major valves. With appropriate sizing and attachment, the polymer valves of the present invention are suitable for replacement of any of the heart valves. Polymer heart valve prostheses for replacement of the mitral and tricuspid valves generally include rigid stents.

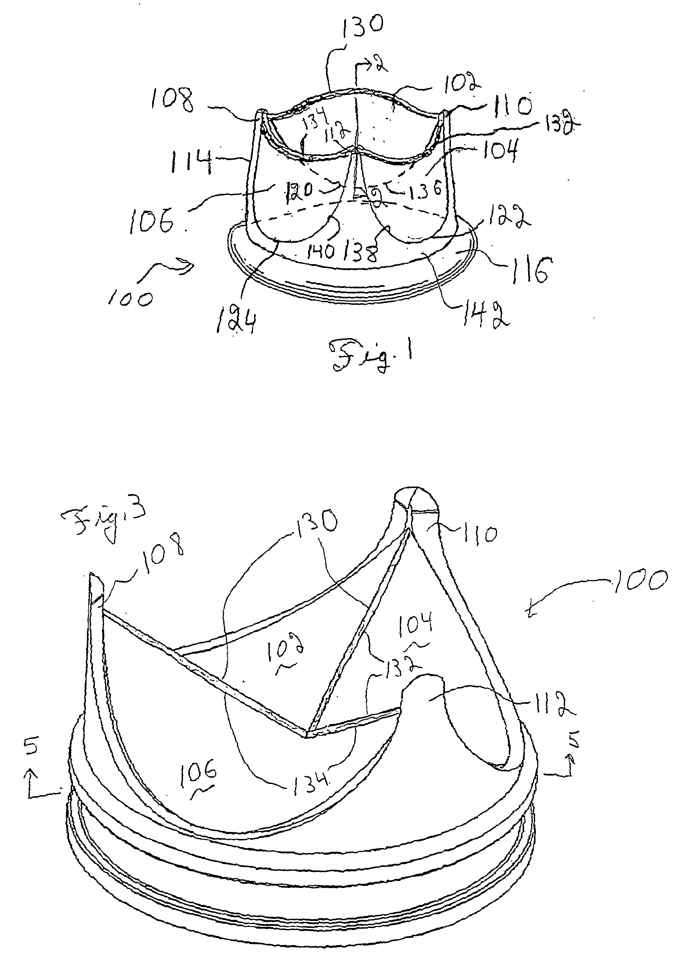

An embodiment of a heart valve prosthesis with flexible polymer leaflets is shown in its fully open position in Fig. 1. Heart valve prosthesis 100 includes leaflets 102, 104, 106, commissure supports 108, 110, 112, support structure/stent 114 and sewing ring 116. Sewing ring 116 is used to attach valve 100 to the patient's tissue annulus with sutures, staples, adhesives or other fastening/attachment mechanisms. Support structure/stent 114 can be relatively rigid, such that the support structure functions as a stent to maintain leaflet function with only attachment to the patient at the base. Alternatively, support structure 114 can be less rigid as part of a stentless valve, with support structure 114 being secured to other anatomical structures to maintain the leaflet function.

Referring to Fig. 1, support structure/stent 114 includes commissure supports 108, 110, 112 and scallops 120, 122, 124 between the commissure supports. Free edges 130, 132, 134 of leaflets 102, 104, 106, respectively, join at the commissure supports 108, 110, 112. Attached edges 136, 138, 140 of leaflets 102, 104, 106 also secure to the support structure along scallops 120, 122, 124. The base of support structure 114 generally is a cylindrical ring 142 that forms the opening into the valve at the upstream end of the valve. Sewing cuff 116 generally is attached to ring 142.

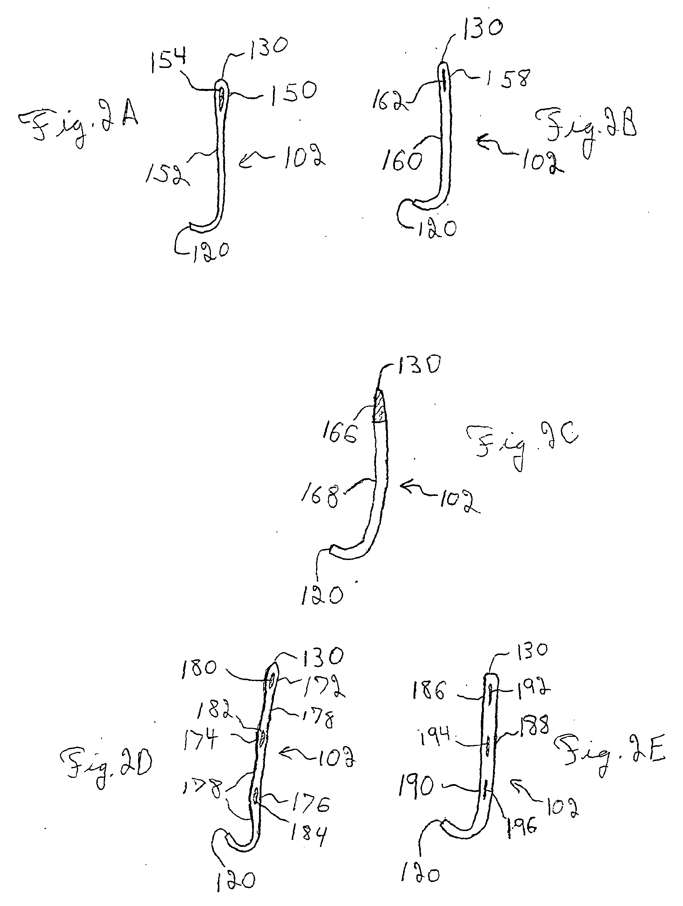

A cross section of nine embodiments of leaflet 102 is shown in Figs. 2A-2I. In the embodiments in Figs. 2A-2C, a single reinforcement is located at or near free edge 130. In the embodiments in Figs. 2D and 2E, there are additional reinforcements besides a reinforcement at or near free edge 130. In the embodiments in Figs. 2F-2I, the reinforcement has the form of a layer of reinforcing material. If a reinforcing layer is used, an additional localized reinforcement with a reinforcing member and/or thickening of the flexible polymer can be used also.

In the embodiment in Fig. 2A, leaflet 102 has a reinforcement 150 near free edge 130. Reinforcement 150 has a thickened cross section relative to body 152 of leaflet 102. Reinforcement 150 can include an optional reinforcing member 154 and/or composition difference to impart additional strength to reinforcement 150 relative to the other portions of leaflet 102. In the embodiment of Fig. 2A, leaflet 102 has a substantially uniform composition and thickness between reinforcement 150 and attached edge 136. In other words, in this embodiment, leaflet 102 is generally uniform except for reinforcement 150 at or near free edge 130. The reinforcement is preferably at least partially within the coaptation region at which adjacent leaflets contact when the valve is closed, as described further below.

Referring to Fig. 2B, leaflet 102 has a reinforcement 158 at or near free edge 130. Reinforcement 158 has a thickness approximately the same as body 160 of leaflet 102. Reinforcement 158 can include a ribbon reinforcing member 162 or other material with increased strength.

Referring to Fig. 2C, reinforcement 166 is produced from a different polymer material than body 168 of leaflet 102. Reinforcement 166 is located at free edge 130 of leaflet 102. In preferred embodiments, reinforcement 166 has a comparable thickness or is thinner than body 168 of leaflet 102. As shown in Fig. 2C, reinforcement 166 is tapered toward free edge 130, although reinforcement 166 can have a uniform thickness. Due to a superior strength of the material in reinforcement 166, it can provide desired levels of tear resistance without added thickness. Reinforcement 166 can include an optional reinforcing member.

In contrast with the embodiments in Figs. 2A-2C, in the embodiments of Figs. 2D and 2E, leaflet 102 has a reinforcement at the free edge 130 of leaflet 102 as well as additional reinforcements in the body of the leaflet. In particular, referring to Fig. 2D, reinforcements 172, 174, 176 have an increased thickness relative to other portions of the body 178 of leaflet 102. Each reinforcement can include an optional reinforcing member 180, 182, 184. The number and orientation of reinforcements away from the free edge and coaptation region can be varied as desired. Referring to Fig. 2E, leaflet 102 has reinforcements 186, 188, 190. Reinforcements 186, 188, 190 can include a reinforcing member 192, 194, 196 and/or composition difference to impart additional strength to the reinforcement relative to the other portions of leaflet 102.

Referring to Fig. 2F, leaflet 102 has a reinforcing layer 200 on flexible polymer layer 202. Even though the reinforcing layer is selected for strength, the reinforcing layer generally should also be flexible. Flexible polymer layer 202 generally imparts desired levels of durability which are enhanced by the presence of the reinforcing layer. As shown in Fig. 2F, reinforcing layer 200 is shown on the inner surface 204 of leaflet 102 directed toward the inflow direction. However, the reinforcing layer can be placed on the outer surface 206 of leaflet 102 directed toward the outflow direction or on both inner surface 204 and outer surface 206.

As shown in Fig. 2F, reinforcing layer 200 covers an entire surface of flexible polymer layer 202. Referring to Fig. 2G, reinforcing layer 208 covers only a portion of inner surface 210 of flexible polymer layer 212. In preferred embodiments, a partial reinforcing layer has an edge at or near free edge 130 or a portion of free edge 130. Again, a partial reinforcing layer can lie on inner surface 210 of flexible polymer layer 212, on outer surface 214 or both surfaces. A partial reinforcing layer preferably covers at least about 50 percent of the surface area of the flexible polymer layer, alternatively at least about 65 percent, in other embodiments at least about 80 percent and, as shown in Fig. 2F, the reinforcing layer can cover essentially an entire surface of the flexible polymer layer.

It may be desirable to place a reinforcing layer within a flexible polymer material. Referring to 2H, a reinforcing polymer layer 220 is sandwiched between flexible polymer layers 222, 224. Thus, the composite material has three layers in a cross section through any portion of leaflet 102.

The reinforcing polymer layer in the embedded/ sandwich version also need not extend through the entire surface of leaflet 102. The reinforcement layer may not extend across the entire length or width of leaflet 102. Referring to Fig. 2I, reinforcing layer 230 only extends through a portion of the length of leaflet 102. Reinforcing layer 230 is embedded within a flexible polymer layer 232. In preferred embodiments, reinforcing layer 230 extends to or near to free edge 130 or a portion of free edge 130. In preferred embodiments, reinforcing layer 230 has an extent through leaflet 102 of at least about 50 percent of the leaflet surface area, alternatively, at least about 65 percent, in other embodiments, at least about 80 percent of the leaflet surface area. Of course, as shown in Fig. 2H, the reinforcing layer can extend through the entire leaflet.

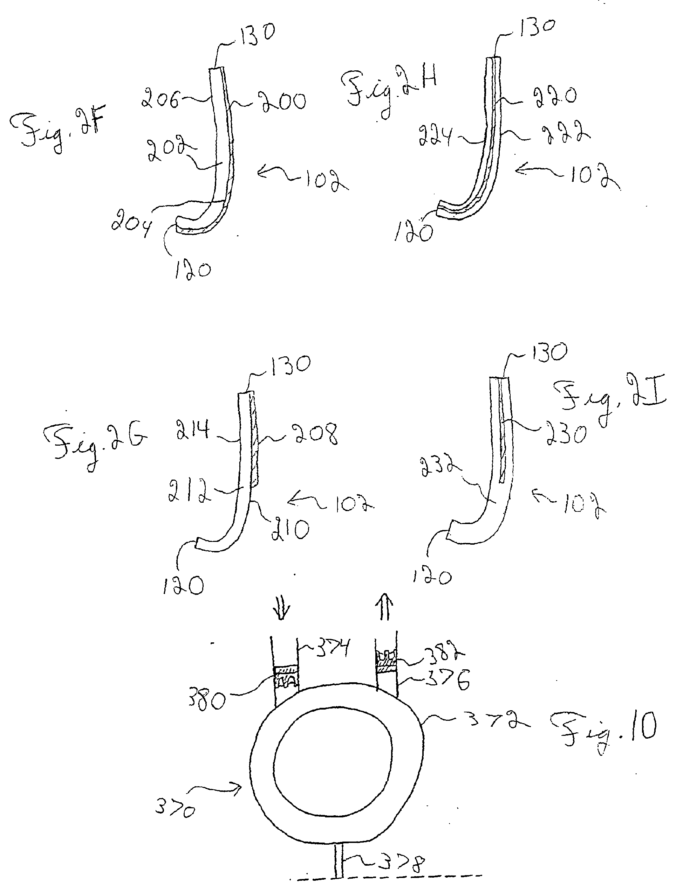

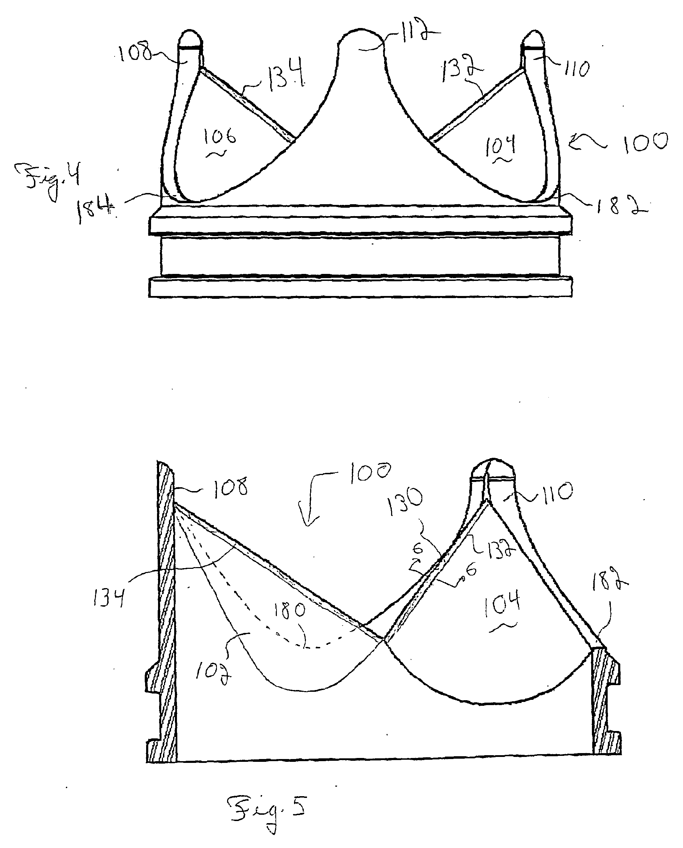

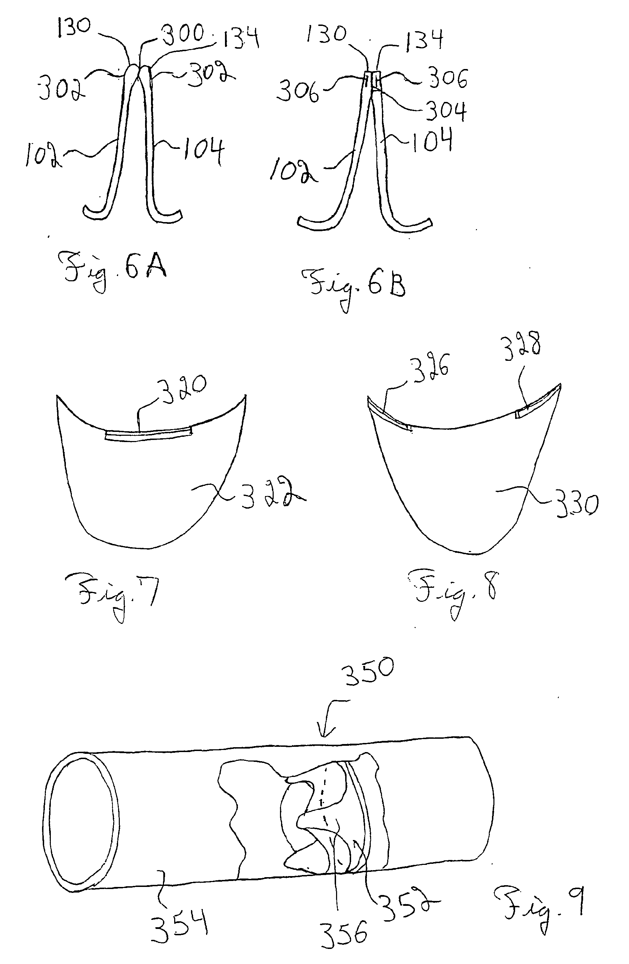

Heart valve prosthesis 100 with closed polymer leaflets is shown in Figs. 3-5. Leaflets 102, 104, 106 contact the respective adjacent leaflets to close the opening through the valve. As shown in Figs. 3-5, leaflets 102, 104, 106 have a single reinforcement at their free edges 130, 132, 134, although other embodiments may have multiple reinforcements. Two embodiments of the coaptation region are shown in Figs. 6A and 6B, respectively. Referring to Fig. 6A, coaptation region 300 is located where reinforcements 302 of adjacent leaflets 102, 104 meet. In this embodiment, the expanded thickness at reinforcements 302 helps to define the coaptation region, as shown in Fig. 6A. In the embodiment shown in Fig. 6B, coaptation region 304 extends over a larger region than the corresponding extent of the reinforcements 306. Similarly, the coaptation region can be smaller than the reinforcement.

As shown in Figs. 1 and 3-5, reinforcements are located at or near the free edge along the entire length of the free edge of the leaflet. However, in other embodiments, the reinforcement only extends over a portion of the free edge. As shown in Fig. 7, reinforcement 320 is located only at the center of the free edge of leaflet 322. In the embodiment of Fig. 8, reinforcements 326, 328 are located near the ends of the free edge of leaflet 330. Additional reinforcements can be included along the free edge of leaflet 322 between reinforcements 326, 328. If the reinforcements do not extend along the entire length of the free edge, the extent of the reinforcement can be selected to yield a desired level and location of tear resistance. If multiple sections of reinforcement are located along the free edge of a single leaflet, the number of reinforcement sections and the length of each section along the free edge can be varied.

The valve prosthesis can be incorporated into a vascular graft with a conduit for replacement of a venous valve or for the replacement of an aortic or pulmonary heart valve. A valved venous prosthesis 350 is shown in a fragmentary view in Fig. 9. Prosthesis 350 includes a three leaflet polymer valve 352 in a conduit 354. Support structure/stent 356 can be rigid or flexible with corresponding appropriate attachment to conduit 354. For example, if support structure/stent 356 is flexible, the leaflet support is attached to the conduit for support. Conduit 354 can be made from natural materials, such as fixed bovine pericardium, or synthetic materials, such as polymers, for example, polyesters.

In addition, a polymer valve as described herein can be incorporated into a left ventricular assist device 370, as shown in Fig. 10. Left ventricular assist devices are implanted devices generally used to maintain the ventricular pumping function of a patient with a damaged or diseased heart awaiting a heart transplant. Left ventricular assist device 370 includes a drive unit 372, an inflow tube 374, an outflow tube 376 and connection 378. Drive unit 372 includes a pump to provide pulsatile flow from inflow tube 374 to outflow tube 376. Connection 378 provides for electrical or pneumatic control signals to be directed to the drive unit from a controller and power supply, generally external to the patient. Inflow tube 374 includes an inflow valve 380, and outflow tube 376 includes an outflow valve 382. Arrows depict the blood flow through inflow tube 374 and outflow tube 376 as controlled by valves 380, 382. Either one or both of inflow valve 380 and outflow valve 382 can be a polymer valve as described herein.

For any of the prosthetic valve embodiments, if the support structure/stent 114 is formed from a rigid material that supports the leaflets, suitable rigid materials include, for example, rigid polymers, metals, ceramics, carbon materials and combinations thereof. Suitable rigid polymers include, for example, polyacetals, such as Delrin® and Celcon®, polysulfones, polyethersulfones, polyarylsulfones, polyetheretherketones, and polyetherimides. Suitable metals include biocompatible metals, such as, stainless steel, titanium, cobalt alloys, such as Elgiloy®, a cobalt-chromium-nickel alloy, and MP35N, a nickel-cobalt-chromium-molybdenum alloy, and Nitinol®, a nickel-titanium alloy. Heart valve stents made from spring metals, such as Elgiloy®, exhibit good mechanical properties, such as strength and fatigue endurance, and can have a smaller cross-section than corresponding polymer stents. Composite metal/polymer heart valve stents are described in WO 0 149 335 A. In addition, stents can be produced from ceramic materials, such as pyrolytic carbon, silicon carbides or metal carbides, hydroxyapatite and alumina. Suitable stents can also be produced from carbons such as graphite. Composites suitable for stents that advantageously combine pyrolytic carbon and carbides are described in WO 0 141 826 A.

Support structures that are flexible can be produced, for example, from flexible polymers or metals. Suitable flexible polymers include, for example, polyurethanes, polydimethyl siloxane and polytetrafluoroethylene. Flexible support structures generally can be produced from the same flexible polymer as the leaflets, a different flexible polymer or a combination thereof. To form the support structure, the flexible polymer can be formed into a sheet, woven into a fabric or produced by a variety of other approaches.

Suitable flexible polymers for support structures also include resorbable polymers, such as, dextran, hydroxyethyl starch, gelatin, derivatives of gelatine, polyvinylpyrrolidone, polyvinyl alcohol, poly[N-(2-hydroxylpropyl) methacrylamide], polyglycols, polyesters, poly (orthoesters), poly(ester amides), and polyanhydrides. Resorbable polyesters include, for example, poly (hydroxy acids) and copolymers thereof, poly($\epsilon$-caprolactone), poly (dimethyl glycolic acid), and poly (hydroxy butyrate). Preferred resorbable polymers include, for example, D, L-polylactic acid, L-polylactic acid, poly(glycolic acid), and copolymers of L-lactic acid, D-lactic acid and glycolic acid. The formation of heart valve stents from resorbable polymers is described further in U.S. Patent 5,728,152 to Mirsch II et al., entitled "Bioresorbable Heart Valve Support".

The leaflets can be formed separately from the support structures, or the leaflets can be formed directly in association with the support structures. If the leaflets are formed separately, they can be attached to the leaflets supports by an approach suitable for the particular materials of the components. For example, the leaflets can be connected to the support by heat bonding, suture, an adhesive or the like. The leaflets can be formed in direct association with the supports whether or not the support is formed from the same material. If the leaflets are formed directly in association with the supports, the support is incorporated into the process for leaflet formation. Leaflet formation is described in the following section.

Sewing cuff 116 generally extends from base 142 of support structure 114. Sewing cuff 116 facilitates the attachment of the heart valve prosthesis to the patient or device. Sutures, staples and/or other fastening mechanisms are passed through the sewing cuff to secure sewing cuff 116 to the patient's tissue annulus or to a conduit prosthesis or the like. Sewing cuff 116 preferably extends outward from base 142 so that the fastening mechanism can be conveniently passed through sewing cuff 116 to attach the valve without significant risk of piercing leaflets 102, 104, 106. Sewing cuff 116 can be produced from natural material, synthetic material or combinations thereof.

Suitable natural materials for sewing cuff 116 include fixed/ crosslinked tissue, such as bovine or porcine pericardial tissue. Crosslinking provides mechanical stabilization, for example, by preventing enzymatic degradation of the tissue. Crosslinking also removes antigenic sites that could result in the patient's rejection of the bioprosthesis. Glutaraldehyde or formaldehyde typically is used for fixation, but other fixatives can be used, such as epoxides, genipin and other difunctional aldehydes.

Suitable synthetic materials include flexible polymers, generally woven into a fabric. Preferred materials include, for example, polyesters, or polytetrafluoroethylene. Fabric sewing cuffs can include antimicrobial metals or agents to reduce the incidence of infection following implantation of the prosthesis into the patient.

Leaflet Structure, Composition and Production

In bioprostheses, flexible leaflets are designed to approximate native leaflet function. While these leaflets are flexible, they must have a well defined and stable configuration to properly close the valve at each cycle to prevent back flow. Also, the leaflets should be durable to provide stable performance over many years of use.

The polymer leaflets flex between a generally fully open position and a generally closed position. In the open position, the free edges of the polymer leaflets form the downstream opening of the valve and do not significantly resist forward blood flow. In the closed position, the free edges of adjacent leaflets contact in the coaptation region to close the valve and do not allow significant leakage.

Suitable polymeric materials for formation into the leaflets include, for example, synthetic polymers as well as purified biological polymers and combinations thereof. Flexible polymers include elastomers and other polymers that can sustain significant flexure, bending, twisting, wear and/or deformation without structural failure. Appropriate synthetic polymers include, without limitation, polyamides (e.g., nylon), polyesters, polyacrylates, vinyl polymers (e.g., polyolefins, polyethylene, polytetrafluoroethylene or other halogenated polymers, polypropylene, ethylene-propylene copolymers, ethylene-propylene-diene monomer copolymer (EPDM) and polyvinylchloride), polycarbonates, polyacetals (e.g., Delrin®), polyurethanes, polydimethyl siloxanes, cellulose acetates, ethylene vinyl acetates, polysulfones, nitrocelluloses, derivatives thereof, similar copolymers, and mixtures thereof. Particularly preferred flexible polymer materials for the formation of flexible polymer heart valve leaflets include, for example, polyurethanes, polydimethyl siloxanes, polytetrafluoroethylenes, derivatives thereof and mixtures thereof.

Biological polymers can be naturally occurring or produced *in vitro* by, for example, fermentation and the like. Purified biological polymers can be appropriately formed into a substrate by techniques such as weaving, knitting, casting, molding, extrusion, cellular alignment and magnetic alignment. Suitable biological polymers include, without limitation, collagen, elastin, silk, keratin, gelatin, polyamino acids, polysaccharides (e.g., cellulose and starch) and copolymers thereof.

Preferred polymers are biocompatible. In preferred embodiments of flexible leaflets, the unreinforced polymer layers generally have a thickness from about 50 microns to about 1000 microns and more preferably from about 100 microns to about 300 microns. A flexible polymer used to form the leaflets of heart valve prostheses is preferably a polymer that has sufficient durability to withstand the repeated cycling required for replacement heart valve use. For a human patient, the valve must cycle about 40 million times each year, and the valve ideally must remain functional over the remaining natural expected lifetime of the patient. Current tissue valves may require replacement following about 400 million to about 600 million cycles. Therefore, the polymer substrate preferably can withstand at least about 400 million cycles and more preferably can withstand more than about 600 million cycles without significant structural deterioration. Only a few classes of currently available flexible polymers are believed capable of such performance requirements. Polyurethanes are one of these classes.

The leaflets can be coated with a thin diamond-like carbon (DLC) coating. The DLC coating is extremely durable, inert and blood compatible. The diamond-like carbon coating provides a barrier that protects the substrate from undesirable reactions with body fluids. Even though the diamond-like carbon is a very hard material, the DLC coating does not affect the flexibility of the coated leaflet. The DLC coating resists cracking even when the polymer is flexed repeatedly. Furthermore, the diamond-like carbon coated polymer is less thrombogenic (more blood compatible) than the uncoated polymer. Preferred approaches for the deposition of a DLC coating on a polymer substrate include, for example, ion beam assisted deposition and radio-frequency plasma deposition. DLC coating of polymer substrates is further described in WO 0 143 790.

As noted above, the polymer leaflets preferably include a reinforcement at or near the free edge of the leaflet in the coaptation region. A reinforcement extends to or near the edge if the reinforcement material extends to within 5 millimeters (mm) of the edge, preferably within 2 mm, and more preferably within 1 mm of the free edge. A free edge is referred to as being reinforced if a reinforcement material is located at or near the free edge. In preferred embodiments, the reinforcement extends along the entire free edge of the leaflet, although the reinforcement may only extend along a portion of the free edge. For example, the reinforcement may only cover the center of the free edge, as shown in Fig. 7, or the portion of the free edge near the commissure supports, as shown in Fig. 8.

The reinforcement can have a thickness less than, equal to, or greater than the body of the leaflet. If the reinforcement is thicker than the body of the leaflet, the additional thickness relative to the leaflet body generally is from about 5 percent to about 100 percent and more preferably from about 20 percent to about 50 percent. Reinforcements with increased thicknesses, such as in Fig. 2A, generally have a width of the thickened reinforcement from about 0.1 millimeters (mm) to about 10 mm and preferably from about 1 mm to about 5 mm. Reinforcements that do not have an increased thickness, as shown in Figs. 2B, 2C and 2E, include another composition within the reinforcement besides the polymer of the leaflet. Reinforcements that have increased thicknesses may or may not include another composition within the reinforcement. Layer reinforcements, such as those shown in Figs. 2F-2I, generally have a thickness from about 0.01 mm to about 1 mm, and preferably from about 0.05 mm to about 0.5 mm.

If a reinforcing member is included, as shown in Figs. 2A, 2B, 2D and 2E, the reinforcing member generally can have any shape as long as the dimensions are appropriate. A single reinforcing member can extend across all three leaflets or multiple reinforcing members can be used. The reinforcing member is preferably a flat ribbon. Flat ribbons are less likely to become separated from the surrounding polymer material. Thus, leaflets reinforced by flat ribbons, such as fabric, are more durable than those reinforced by rounded reinforcing members. The ribbon preferably has a width from about 0.1 mm to about. 10 mm, and more preferably from about 1 mm to about 5 mm. If the width of the reinforcing member is too small, it also can separate from the polymer. In addition, the reinforcing member generally has a thickness from about 0.01 mm to about 0.5 mm and more preferably from about 0.03 to about 0.3 mm.

If a reinforcing layer is used, the leaflet generally includes a second composition as the reinforcing layer, in addition to the flexible polymer forming the leaflet, although in the embodiments of Figs. 2G and 2I, the reinforcement can involve just a thickened region with the flexible polymer. As shown in Figs. 2F-2I, the leaflets can have an increased thickness due to the reinforcing layers. The reinforcing layer material and the flexible polymer material can be intertwined such that there are no clear layers formed by the two materials. This intertwining results if the reinforcing layer is not formed from a solid sheet of material. Similar intertwining can result also with the use of a reinforcing member that is more localized in placement but, nevertheless is not formed from a solid sheet of material.

If the reinforcement, such as a reinforcing member or reinforcing layer, includes a different material from the remaining portion of the leaflet, suitable reinforcement materials increase the strength of the leaflet and include, for example, other polymers, polymer-ceramic composites, such as fiberglass, metal, carbon materials, and combinations thereof. Suitable metals include, for example, biocompatible metals, such as, stainless steel, titanium, cobalt alloys, such as Elgiloy®, a cobalt-chromium-nickel alloy, and MP35N, a nickel-cobalt-chromium-molybdenum alloy, and Nitinol®, a nickel-titanium alloy. Suitable carbon materials include, for example, graphite fibers. Generally, if a different polymer is introduced for reinforcement, the additional reinforcing polymer would be a stronger polymer than the polymer used for the leaflet body, although the same types of polymers as those listed above for use in the leaflets are suitable. The additional polymer can be a more crosslinked version of the polymer in the leaflet or a similar polymer with a different monomer composition or polymeric chain length, to introduce additional strength. Preferred reinforcing polymers include, for example, polyesters, poly-tetrafluoro-ethylene and polyparaphenylene terephtha-lanlide (KEVLAR®).

If the reinforcement, either reinforcing member or reinforcing layer, are formed with a reinforcing material that is different from the flexible polymer forming the majority of the leaflet structure, the reinforcing material can be in the form of a solid material or a porous material. The use of a porous material provides for intertwining of the reinforcing material and the remaining flexible polymer forming the leaflet. The porous material can be formed by weaving, knitting or perforating the reinforcing material that is subsequently combined with the flexible polymer to form the leaflet. In some preferred embodiments, the reinforcing material is in the form of a woven or knitted fabric. The resulting structure is a polymer sealed fabric. The use of a fabric provides for the formation of an very stable structure that can also provide an increase in strength.

Preferred reinforcing materials are flexible, such that they do not significantly affect the valve performance. Specifically, in preferred embodiments, a reinforced section is no more than about 3 times as rigid as an unreinforced section, preferably no more than about 2 times as rigid and more preferably no more than about 1.5 times as rigid, where flexural rigidity is measured by the bending modulus. The ratio of flexural rigidities of the reinforced leaflet divided by the unreinforced leaflet can be calculated from the ratios of Young's moduli and the thicknesses.

In particular,

$$R = (H_2/H_0)^3 + (E_1/E_0 - 1) \times (H_1/H_0)^3,$$

where R is the ratio of the flexural rigidity of the reinforced polymer leaflet divided by the flexural rigidity of the unreinforced polymer leaflet. $E_1/E_0$ is the ratio of the Young's modulus of the reinforcing material divided by the Young's modulus of the unreinforced polymer. $H_0$ is the thickness of the unreinforced leaflet, $H_2$ is the thickness of the reinforced leaflet, and $H_1$ is the thickness of the reinforcing material. The Young's modulus can be evaluated by a procedure similar to that described in ASTM D882, entitled Standard Test Method for Tensile Properties of Thin Plastic Sheeting.

The leaflets can be formed from the selected polymer by molding, weaving, extruding, dip coating or casting the polymer into appropriate forms. Molding processes can be performed with or without inclusion of a separate support structure/stent within the mold. Preferred methods include casting and dip coating.

To perform dip coating, a mandrel is dipped into a solvent solution of the desired polymer or into a heated polymer melt. The mandrel is machined to have the desired shape of the polymer leaflet. A support structure/stent can be placed over the mandrel prior to dip coating to obtain a coating directly over the support structure. Alternatively, a support structure can be formed on the mandrel from the same polymer as the leaflets during the dip coating process. In other alternative embodiments, the leaflets can be associated with the support structure/stent following removal of the leaflets from the mandrel.

The leaflets and support structures are removed from the mandrel following completion of the coating process. The coating thickness can be varied by changing the temperature of the melt and/or polymer concentration of the solution. Upon cooling and/or evaporation of the solvent, the polymer structure is formed on the mandrel. Multiple dipping steps can be used if desired.

To form the reinforcement in a molding process, the mold can be altered to provide a thickened portion of the leaflet at the reinforcement. Alternatively, a reinforcement material can be placed in the mold at the location of the reinforcement such that the polymer forms the leaflet around the reinforcement material. In a dip coating process, the shape of the mandrel can be varied to provide a thickened edge, or the free edge can be dipped additional times to provide a thickened portion at that edge. Alternatively, a reinforcement material can be placed along the mandrel either at the start of the dip coating process or after an initial polymer coating has been formed. If one or more reinforcement members are used, individual reinforcement members can be placed on the mandrel to extend through all of the leaflets.

The polymer reinforcement layers shown in Figs. 2F-2I can be made by placing the reinforcement material within a mold or on a mandrel similar to other reinforcements. If the reinforcing polymer layer is embedded in a flexible polymer, the leaflet can be made in layers to facilitate the fabrication. If a mandrel is used, the reinforcing polymer layer can be applied by dip coating a layer with a different composition onto another layer.

Formation of Prostheses

After the leaflets are formed, additional processing steps may be needed to complete the production of the prosthesis. First, if the leaflets were not formed directly in association with the support structure/stent, the leaflets are connected to the support structure. Any additional structures, such as a sewing cuff, are connected to the support structure. Sewing cuffs and the like generally are added at or near the inflow edge. If the valve is incorporated into a conduit, the conduit can be connected to or formed around the valve such that the valve is securely connected to the conduit. Suture, staples, adhesive, and other fastening mechanisms and combinations thereof can be used to connect the support structures to the other components.

Packaging, Distribution and Use

For distribution, the medical devices are placed in sealed and sterile containers. The containers can be dated such that the date reflects the maximum advisable storage time, if components of the medical device should not be stored indefinitely. The containers are packaged along with instructions for the proper use and/or implantation of the medical device and along with other appropriate and/or required labeling. The containers are distributed to health care professionals for use in appropriate medical procedures, such as implantation of a prosthesis and the like. Heart valve prostheses and valved vascular prostheses can be implanted, for example, using standard surgical procedures.

The embodiments described above are intended to be illustrative and not limiting. Additional embodiments are within the claims. Although the present invention has been described with reference to preferred embodiments, workers skilled in the art will recognize that changes may be made in form and detail without departing from the scope of the invention.

**Claims**

1. A valve prosthesis (100) comprising a support structure (114) with a plurality: of commissure supports (108, 110, 112) and a plurality of flexible polymer leaflets (102, 104, 106) extending between the commissure supports and having a coaptation region, **characterised in that** the leaflets have a reinforced free coaptation edge (130, 132, 134) comprising a reinforcement comprising a material that is different from the polymer leaflet.

2. The valve prosthesis of claim 1, wherein the reinforcement comprises a polymer.

3. The valve prosthesis of claim 1, wherein the reinforcement comprises metal.

4. The valve prosthesis of claim 1, wherein the reinforcement comprises a fabric.

5. The valve prosthesis according to any preceding claim, wherein the reinforcement has a flexural rigidity no more than a factor of three greater than the unreinforced portions of the leaflet.

6. The valve prosthesis according to any preceding claim, wherein the reinforcement is embedded within the flexible polymer leaflets.

7. The valve prosthesis according to claims 1 to 5, wherein the reinforcement is on the surface of the flexible polymer leaflets.

8. The valve prosthesis according to any preceding claim, wherein the reinforcement extends along the entire reinforced free coaptation edge.

9. The valve prosthesis according to claims 1 to 7, wherein the reinforcement extends along a portion of the reinforced free coaptation edge.

10. The valve prosthesis according to any preceding claim, wherein the reinforcement comprises a reinforcing member.

11. The valve prosthesis of claim 10, wherein the reinforcing member comprises a flat ribbon.

12. The valve prosthesis of claim 10 and 11, wherein the reinforcing member has a width no larger than the extent of the coaptation region.

13. The valve prosthesis of any of claims 10 and 11, wherein the reinforcing member has a width at least as large as the extent of the coaptation region.

14. The valve prosthesis of claims 10 to 13, wherein the reinforcing member has a thickness of 0.01 mm to about 0.5 mm.

15. The valve prosthesis according to claims 1 to 9, wherein the reinforcement comprises a reinforcing layer.

16. The valve prosthesis of claim 15, wherein the reinforcing layer extends at least 50 percent of the area covered by the leaflets.

17. The valve prosthesis according to any preceding claim, wherein the flexible polymer leaflets further comprise reinforcements away from the copatation region.

18. The valve prosthesis according to any preceding claim, wherein the polymer leaflets having a reinforced free coaptation edge comprise a polymer selected from the group consisting of polyamides, polyesters, polyacrylates, polyethylene, polytetrafluoroethylene, polypropylene, ethylene-propylene copolymers, ethylene-propylenediene monomer copolymer, polyvinylchloride, polycarbonates, polyacetals, polyurethanes, polydimethyl siloxanes, cellulose acetates, ethylene vinyl acetates, polysulfones, nitrocelluloses, and derivatives, mixtures and copolymers thereof.

19. The valve prosthesis according to any preceding claim, wherein the polymer leaflets having a reinforced free coaptation edge have a substantially uniform composition and thickness over the flexible body of the leaflet between the support structure and the reinforced free coaptation edge.

20. The valve prosthesis according to any preceding claim, wherein the reinforcement further comprises a thickened portion of polymer forming the body of the polymer leaflets.

21. The valve prosthesis of any preceding claim, wherein the support structure comprises a flexible material.

22. The valve prosthesis of claim 21, wherein the support structure comprises the same polymer as the leaflet

23. The valve prosthesis of any of claims 1-20, wherein the support structure comprises a rigid material.

24. An artificial heart comprising a valve prosthesis of any preceding claim.

**Patentansprüche**

1. Klappenprothese (100), die eine Tragstruktur (114) mit einer Mehrzahl von Kommissurenträgern (108, 110, 112) und einer Mehrzahl von flexiblen Polymerflügeln (102, 104, 106) umfasst, die zwischen den Kommissurenträgern verlaufen und eine Koaptationsregion aufweisen, **dadurch gekennzeichnet, dass** die Flügel eine verstärkte freie Koaptationskante (130, 132, 134) haben, die eine Verstärkung umfasst, die aus einem Material besteht, das sich von dem Polymerflügel unterscheidet.

2. Klappenprothese nach Anspruch 1, wobei die Verstärkung aus einem Polymer besteht.

3. Klappenprothese nach Anspruch 1, wobei die Verstärkung aus Metall besteht.

4. Klappenprothese nach Anspruch 1, wobei die Verstärkung aus einem Stoff besteht.

5. Klappenprothese nach einem der vorherigen Ansprüche, wobei die Verstärkung eine Biegesteifigkeit hat, die um nicht mehr als einen Faktor von drei größer als bei den nicht verstärkten Abschnitten ist.

6. Klappenprothese nach einem der vorherigen Ansprüche, wobei die Verstärkung in den flexiblen Polymerflügeln eingebettet ist.

7. Klappenprothese nach den Ansprüchen 1 bis 5, wobei die Verstärkung auf der Oberfläche des flexiblen Polymerflügels vorliegt.

8. Klappenprothese nach einem der vorherigen Ansprüche, wobei die Verstärkung entlang der gesamten verstärkten freien Koaptationskante verläuft.

9. Klappenprothese nach den Ansprüchen 1 bis 7, wobei die Verstärkung entlang einem Abschnitt der verstärkten freien Koaptationskante verläuft.

10. Klappenprothese nach einem der vorherigen Ansprüche, wobei die Verstärkung ein Verstärkungselement umfasst.

11. Klappenprothese nach Anspruch 10, wobei das Verstärkungselement ein flaches Band umfasst.

12. Klappenprothese nach Anspruch 10 und 11, wobei das Verstärkungselement eine Breite von nicht mehr als dem Umfang der Koaptationsregion hat.

13. Klappenprothese nach einem der Ansprüche 10 und 11, wobei das Verstärkungselement eine Breite hat, die wenigstens dem Umfang der Koaptationsregion entspricht.

14. Klappenprothese nach den Ansprüchen 10 bis 13, wobei das Verstärkungselement eine Dicke von 0,01 mm bis etwa 0,5 mm hat.

15. Klappenprothese nach den Ansprüchen 1 bis 9, wobei die Verstärkung eine Verstärkungsschicht umfasst.

16. Klappenprothese nach Anspruch 15, wobei sich die Verstärkungsschicht über wenigstens 50 Prozent der von den Flügeln abgedeckten Fläche erstreckt.

17. Klappenprothese nach einem der vorherigen Ansprüche, wobei die flexiblen Polymerflügel ferner Verstärkungen entfernt von der Koaptationsregion aufweisen.

18. Klappenprothese nach einem der vorherigen Ansprüche, wobei die Polymerflügel, die eine verstärkte freie Koaptationskante aufweisen, ein Polymer beinhalten, das ausgewählt ist aus der Gruppe bestehend aus Polyamiden, Polyestern, Polyacrylaten, Polyethylen, Polytetrafluorethylen, Polypropylen, Ethylen-Propylen-Copolymeren, Ethylen-Propylendien-Monomer-Copolymer, Polyvinylchlorid, Polycarbonaten, Polyacetalen, Polyurethanen, Polydimethylsiloxanen, Celluloseacetaten, Ethylenvinylacetaten, Polysulfonen, Nitrocellulosen und Derivaten, Gemischen und Copolymeren davon.

19. Klappenprothese nach einem der vorherigen Ansprüche, wobei die Polymerflügel mit einer verstärkten freien Koaptationskante eine im Wesentlichen einheitliche Zusammensetzung und Dicke über den flexiblen Körper des Flügels zwischen der Tragstruktur und der verstärkten freien Koaptationskante aufweisen.

20. Klappenprothese nach einem der vorherigen Ansprüche, wobei die Verstärkung ferner einen verdickten Abschnitt aus Polymer beinhaltet, der den Körper der Polymerflügel bildet.

**21.** Klappenprothese nach einem der vorherigen Ansprüche, wobei die Tragstruktur ein flexibles Material umfasst.

**22.** Klappenprothese nach Anspruch 21, wobei die Tragstruktur das gleiche Polymer wie der Flügel umfasst.

**23.** Klappenprothese nach einem der Ansprüche 1-20, wobei die Tragstruktur ein steifes Material umfasst.

**24.** Künstliches Herz, das eine Klappenprothese nach einem der vorherigen Ansprüche umfasst.

## Revendications

**1.** Prothèse valvulaire (100) comprenant une structure de support (114) à pluralité de supports de commissures (108, 110, 112) et une pluralité de feuillets en polymère souples (102, 104, 106) s'étendant entre les supports de commissures et ayant une région de coaptation, **caractérisée en ce que** les feuillets ont un bord de coaptation libre renforcé (130, 132, 134) comprenant un renforcement comprenant une matière qui est différente du feuillet en polymère.

**2.** Prothèse valvulaire selon la revendication 1, dans laquelle le renforcement comprend un polymère.

**3.** Prothèse valvulaire selon la revendication 1, dans laquelle le renforcement comprend un métal.

**4.** Prothèse valvulaire selon la revendication 1, dans laquelle le renforcement comprend un tissu.

**5.** Prothèse valvulaire selon l'un quelconque des revendications précédentes, dans laquelle le renforcement a une rigidité à la flexion qui ne dépasse pas par plus d'un facteur de trois celle des parties non renforcées du feuillet.

**6.** Prothèse valvulaire selon l'un quelconque des revendications précédentes, dans laquelle le renforcement est noyé dans les feuillets en polymère souples.

**7.** Prothèse valvulaire selon l'un quelconque des revendications 1 à 5, dans laquelle le renforcement se trouve sur la surface des feuillets en polymère souples.

**8.** Prothèse valvulaire selon l'un quelconque des revendications précédentes, dans laquelle le renforcement s'étend tout le long du bord de coaptation libre renforcé.

**9.** Prothèse valvulaire selon l'un quelconque des revendications 1 à 7, dans laquelle le renforcement s'étend sur une partie du bord de coaptation libre renforcé.

**10.** Prothèse valvulaire selon l'un quelconque des revendications précédentes, dans laquelle le renforcement comprend un élément de renforcement.

**11.** Prothèse valvulaire selon la revendication 10, dans laquelle l'élément de renforcement comprend un ruban plat.

**12.** Prothèse valvulaire selon les revendications 10 et 11, dans laquelle l'élément de renforcement a une largeur qui ne dépasse pas l'étendue de la région de coaptation.

**13.** Prothèse valvulaire selon l'une quelconque des revendications 10 et 11, dans laquelle l'élément de renforcement a une largeur au moins aussi grande que l'étendue de la région de coaptation.

**14.** Prothèse valvulaire selon les revendications 10 à 13, dans laquelle l'élément de renforcement a une épaisseur de 0,01 mm à 0,5 mm.

**15.** Prothèse valvulaire selon les revendications 1 à 9, dans laquelle le renforcement comprend une couche de renforcement.

**16.** Prothèse valvulaire selon la revendication 15, dans laquelle la couche de renforcement s'étend sur au moins 50% de la zone couverte par les feuillets.

**17.** Prothèse valvulaire selon l'une quelconque des revendications précédentes, dans laquelle les feuillets en polymère souples comprennent en outre des renforcements éloignés de la région de coaptation.

**18.** Prothèse valvulaire selon l'une quelconque des revendications précédentes, dans laquelle les feuillets en polymère ayant un bord de coaptation libre renforcé comprennent un polymère sélectionné dans le groupe consistant en polyamides, polyesters, polyacrylates, polyéthylène, polytétrafluoroéthylène, polypropylène, copolymères éthylène-propylène, copolymère éthylène-monomère de propylenediène, chlorure de polyvinyle, polycarbonates, polyacétals, polyuréthanes, siloxanes polydiméthyliques, acétates de cellulose, copolymères éthylène/acétate de vinyle, polysulfones, nitrocelluloses, et dérivés, mélanges et copolymères de ceux-ci.

**19.** Prothèse valvulaire selon l'une quelconque des revendications précédentes, dans laquelle les feuillets en polymère ayant un bord de coaptation libre renforcé ont une composition et une épaisseur essentiellement uniformes sur le corps souple du feuillet entre la structure de support et le bord de coaptation libre renforcé.

**20.** Prothèse valvulaire selon l'une quelconque des revendications précédentes, dans laquelle le renforcement comprend en outre une partie épaissie de polymère formant le corps des feuillets en polymère.

**21.** Prothèse valvulaire selon l'une quelconque des revendications précédentes, dans laquelle la structure de support comprend une matière souple.

**22.** Prothèse valvulaire selon la revendication 21, dans laquelle la structure de support comprend le même polymère que le feuillet.

**23.** Prothèse valvulaire selon l'une quelconque des revendications 1 à 20, dans laquelle la structure de support comprend une matière rigide.

**24.** Coeur artificiel comprenant une prothèse valvulaire selon l'une quelconque des revendications précédentes.

Fig. 1

Fig 3

Fig. 2A

Fig. 2B

Fig. 2C

Fig. 2D

Fig. 2E

Fig. 2F

206 — 200

202 —

204

130

— 102

120

Fig. 2H

224 — 220

222

— 102

120

130

Fig. 2G 214 — 208

212 —

210

120

130

— 102

Fig. 2I

230

232

— 102

120

130

374

382

376

380

372 Fig. 10

370

378

Fig. 4

Fig. 5

Fig. 6A

Fig. 6B

Fig. 7

Fig. 8

Fig. 9